(19)

Europäisches
Patentamt
European
Patent Office
Office européen
des brevets

(11) **EP 2 438 880 A1**

(12) **EUROPEAN PATENT APPLICATION**

(43) Date of publication:
**11.04.2012 Bulletin 2012/15**

(51) Int Cl.:
***A61B 19/00*** *(2006.01)*

(21) Application number: **10186627.5**

(22) Date of filing: **05.10.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Universität Bern**
**3012 Bern (CH)**

(72) Inventor: **The designation of the inventor has not yet been filed**

(74) Representative: **Schmauder & Partner AG**
**Patent- & Markenanwälte VSP**
**Zwängiweg 7**
**8038 Zürich (CH)**

(54) **Image projection system for projecting image on the surface of an object**

(57)    The invention relates to an image projection system, with a database (2) for storing and retrieving multi-dimensional data of an object and/or models thereof, a navigation system (4, 4.1, 4.3, 40) for determining positional data reflecting the relative placement of an image projector (3) and the object (1), and a rendering engine (5) for generating rendered image data based on multi-dimensional data and/or models thereof and positional data, wherein the image projector (3) is arranged to project the rendered image data as a visible image (1.3) onto the surface of the object (1). A refreshing module (6) is arranged for generating a refreshing event, in order to activate the rendering engine(5) and to refresh the rendered image data.

**Fig. 1**

EP 2 438 880 A1

## Description

The invention relates to an image projection system, comprising:

a) a navigation system for determining positional data reflecting the relative placement of an image projector and an object,

b) a database for storing and retrieving of multidimensional data of an object and/or models thereof, and

c) a rendering engine for generating rendered image data based on the multidimensional data of the object and/or models thereof and positional data, wherein the image projector is arranged to project the rendered image data as a visible image onto the surface of the object.

Moreover, the invention relates to a corresponding image projection method.

## Background Art

Currently available surgical navigation systems utilize registered computer-generated 3D patient specific models to display a virtual scene of the surgical procedure on conventional (TV, LCD etc) screen, thus guiding surgeons on a nearby screen. Whilst such systems have proven to assist in the definition and conduct of surgical procedures and the identification of critical structures, limitations in field of view and lack of intuitiveness have called for the development of alternative visual guidance methods and tools. Augmented reality, the superimposition of 3D computer-generated objects onto real images or video, has been used somewhat successfully at providing a more intuitive view of the surgical scene. Combining the surgeons real world view with the view of 3D computer generated navigation models on a single image allowed surgeons to essentially view structures through overlying tissues. Due to complex registration requirements, this technique was employed primarily in surgeries involving relatively fixed workspaces static anatomical structures such as neurosurgery. Some systems reported the first case of augmented reality use in general soft tissue surgery however, the registration and image merging process were elaborate and time consuming and no accuracy evaluation was provided. Removing the need for the surgeon to divide his line of sight between the patient and image display, and to improve the field of view, a system has been developed for volumetric image overlay, based on a semi transparent mirror, which may be also called image projection system. The device allowed surgeons to view the patient and computer generated 3D models in a single view by projecting models onto the semi transparent mirror placed above the patient, giving the illusion of a 3D model floating immediately above the patient.

More recently, systems have been described, utilizing an image projection system, whereby 3D images were superimposed on the patient's actual body surface during gastrointestinal, hepatobiliary and pancreatic surgery. The projection system - a conventional office like LCD projector - was placed in a fixed and predefined position relative to the patient.

In US 2002/0077533 a visualization device is disclosed for the visualization of data that relate to a medical intervention, wherein data is projected onto the body surface of the patient in the region of the intervention. Data may relate to the position and orientation of intracorporeally guided instruments, based on a sender unit and a navigation system. These data are projected as geometrical shape or figure, for example a circle and/or as arrow, in a specific relationship to the position of the surgical instrument. The geometrical shape on the body is located on the connecting line between the position of the instrument and the line of vision of the surgeon, wherein markings attached to the head of the surgeon are acquired with a stereo camera. Medical image data such as real-time ultrasound or x-ray image data may be projected onto the body.

US 6,314,311 relates to image guided surgery. Prior to a medical procedure, 3D diagnostic image data of a subject is generated by computed tomography scanners, magnetic resonance imaging or other imaging equipment. An image representation of the subject is reconstructed from the data and projected on the subject. The depicted image is adjusted to compensate position and orientation of the subject, either by adjusting the position of the projector or the subject. Optionally, the adjustment is accomplished by adjusting the position and orientation of the subject support in order to achieve a high degree of registration accuracy. The spatial coordinates of the projector, the subject and other system components are monitored or controlled relative to one another or a fix point in space. Appropriate corrections are made to the image so that proper registration is achieved. A contour processor makes corrections to account for the surface contours of the subject. The image representation takes the form of a planar slice, arrays of planar slices, surface renderings, 3D volume renderings, and the like. The projector includes a laser and mirrors which reflect the laser beam. The projector is installed remote and immobile (i.e. on the ceiling), in a predefined and stable geometric configuration to the surgical site and projects the image inside the sterile region without compromising sterility. The tracking system includes a detection unit having receivers such as CCD arrays, IR cameras, or the like and detects passive or active radiation, in order to resolve spatial location of the emitters, which are affixed to the patient support, the projector, or to a surgical tool. An image data processor is arranged in order to reconstruct an image representation of the subject from the image data. Contouring means are provided for adjusting the image representation so that it is undistorted when projected onto a contoured surface of the subject.

The benefits of augmented reality and image overlay techniques are evident. It was concluded that image overlay assisted in the three dimensional understanding of anatomical structures leading to significant beneficial surgical outcomes resulting from reductions in operation time, less frequent intraoperative injuries and reduced bleeding. Augmented reality was found to aid in the determination of correct dissection planes and the localization of tumours, adjacent organs and blood vessels. It has been predicted that such technology could be used to avoid injury to invisible structures and to minimize the dissection and resection of neighbouring tissues.

Despite such benefits, the many identified limitations and lack of validation has prevented any one technique from becoming an accepted addition to current surgical navigation techniques. Some systems recorded registration times of approximately 5 minutes, making the repetition of the registration process during the procedure as organs move, impractical, limiting use to preoperative planning or to static structures.

Moreover, some systems which are commercially available, remain in limited use due to their high cost, intrusiveness and limited work space.

Some systems presented a more cost effective and less intrusive alternative to other systems but the need for an installed LCD projector at a known distance above the patient resulted in little improvement in required set up and registration time. Additionally, to provide a correct overlay of an image modality onto some real world object, the spatial position of the viewpoint (i.e. the user) has to be known in order to cope for parallaxes displacement. To detect the user's position in space is challenging, especially in a surgical environment and methods using tracked hats have been described.

In current image projection systems, the projector is installed remote and immobile relative to the surgical site, such that sterility is not compromised. Accordingly, the task of projecting an image on a different area requires the time consuming steps of moving and adjusting the projector and/or the subject support and thereafter of initiating the generation of a new image based on the position of the projector and/or the subject.

## Summary of the invention

It is the object of the invention to create an image projection system and an image projection method pertaining to the technical field initially mentioned, which enables an easy to use and versatile projection of an image on the surface of an object.

The solution of the invention is specified by the features of claim 1. According to the invention, a refreshing module is arranged for generating a refreshing event, in order to activate the rendering engine and to refresh the rendered image data.

Refreshing events may be generated depending on the current environment and needs. For example, a pushbutton may be arranged on a surface of the projector, which generates a refreshing event when pushed. After adjusting the projector to a new position, the pushbutton may be pushed and rendered image data therefore refreshed. The pushbutton enables an easy to use and versatile projection of the image corresponding to the new position of the projector.

Multidimensional data of the object may relate to any known measurement technique for acquiring tomography multidimensional measurement data of an object, namely to CT (CT: computer tomography or X-ray tomography) data, MRI (MRI: magnetic resonance imaging or nuclear magnetic resonance) data, PET (PET: positron emission tomography) data, seismic tomography data, ultrasound transmission tomography data, electrical or optical impedance tomography data, or any other tomography data. Alternatively, image data acquired by projection techniques (i.e. fluoroscopy, surface scanning etc.) can be used for projection.

Alternatively, multidimensional data may relate to constructional data of the object, like the location of cables, tubes, or any other element in a car, in a building or any other object.

The multidimensional data may be stationary or time-dependent. Time-dependent multidimensional data may be generated in real time by using a corresponding acquiring system.

The navigation system determines positional data reflecting the relative placement of the image projector and the object. Particularly, the navigation system may be designed as a pose measurement system, wherein a combined measurement of the location and the orientation is performed. The pose measurement integratedly measures in real time the spatial pose of the projector, the target object and other objects (instruments, users, tables). The pose measurement of an object is achieved by means of optical tracking (video or marker based), electromagnetic or mechanical tracking. Thus the objects to be tracked are comprised of suitable reference structures (retroreflective spheres, LED, coils). Out of the relative pose of the projector and the target object relative to the pose measurement system, the relative pose between the projector and the target object can be determined.

The navigation system, particularly the pose measurement system may comprise or incorporate any other known measurement technique, for example radio navigation, radar navigation, or satellite navigation.

The rendering engine may comprise any known steps for rendering multidimensional data. For example, in a first step the multidimensional data is segmented into relevant objects and structures, like, for example, tissue, muscle, vessels, tumors. In a second step, a perspective view of the segmented multidimensional data is generated, such that the relevant objects and structures can be easily identified in the image. For example, different colors and/or textures may be used for different objects and structures. In a third step, the image is transformed and distorted in order to be projected onto a surface of the target object, such that the image can be viewed in an undistored manner also in case of complicated object surfaces, like for example the surface of a internal or external parts of the body.

In a preferred embodiment, a timer module generates refreshing events at regular time intervals, preferably at least 20 times a second (20 Hz is a frame rate near that of the human visual perception). This enables a real-time use of the projector, such that the image projected on the target object is always corresponding with the relative placement of the projector and the object. The resulting time lag in the projection, caused by the necessary steps of pose measurement, rendering and data transfer to the projector should be minimal in order to allow for an immersive behaviour of the projection system.

Alternatively, the refreshing events are generated at only a fraction of the perception rate of the human eye (for example only once a second). Thus, current rendered image data are replaced by refreshed rendered image data at discrete points in time. As soon as the difference between current rendered image data and refreshed rendered image data rises above a threshold, the refreshing events are generated more frequently, for example twice a second. The difference between the current rendered image data and the refreshed rendered image data is further monitored, such that the rate of the refreshing events can be increased or decreased accordingly. If required, a first and a second threshold may be defined, wherein the rate of the refreshing events is decreased when the difference is below the first threshold and wherein the rate of the refreshing events is increased when the difference is above the second threshold. The first threshold may be smaller than the second threshold.

Preferably, a position comparator module generates the refreshing event upon the detection of a change in the relative placement of the image projector and the object. Thus, the visible image projected on the surface of the object is refreshed as soon as the projector and/or the object are moved. Hence, the visible image corresponds always to the current relative placement of the projector and the object. When a user of the projector moves it around the object, the internal structure of the object may therefore be investigated from different sides of the object.

Alternatively, a refreshing event is generated only in case of change in the relative pose or placement of the image projector and the object exceeds a threshold. The threshold may be selectable by the user. Thus, if the change in position is below the threshold, the workload on the rendering engine may be decreased.

In a preferred embodiment, a tomography equipment is arranged in order to acquire multidimensional measurement data of an organ, a limb or any other part of a human body, wherein the image projector is arranged to project the visible image on the organ, the limb respectively the other part of a human body measured by the tomography equipment. Any tomography equipment may be used, as described above, whereby many of them are already widely available in current hospitals. Hence, 3D data of an organ of a patient, for example the liver, may be acquired during examination of the patient. If the examination results that according to the 3D data a medical treatment of the patient is required, a surgery procedure may be initiated. During the surgery procedure, a visible image may be projected on the surface of the organ of the patient, e.g. the liver, in order to give the surgeon a further guidance in order to perform the surgery more efficiently.

Alternatively, the multidimensional data is based on construction plans, for example based on 3D computer aided design (CAD) data, of an industrial object like a car, a television or any other product, of a building or of any other object. For example, in a production line, after an assembly step, a visible image may be displayed on the surface of the object in order to guide a person responsible for quality control to critical assembled parts that have to be controlled. Or in a step of providing an opening in the surface of a wall or car roof, for example, a person may be guided such that cables or tubes beneath the surface of the wall or car roof are not damaged or destroyed.

In a preferred embodiment, the image projector is installed in a portable, handheld housing, which preferably is covered by a sterile drape. A portable, handheld housing may be easily moved around an object in order to investigate the object from various sides. Hence, a surgeon may investigate an organ of a patient, e.g. a liver, from various sides in order to find out the optimal surgery procedure. Furthermore, a movable image projector allows for illuminating all accessible surfaces even of complex three-dimensional objects, unobstructed by shadow casts. The portable, handheld housing provides for a mobile, hand held, non stationary and versatile image projector, which may be connected through a cable or through a wireless interface to a control computer, capable of projecting image content through an LCD screen, a color laser system or the like. The image projector may be of a specific size, form and weight allowing it to be moved arbitrarily in space near and around a target object by force of a human hand only.

Alternatively, the projector is attached to a stand, float arm, a cantilever or any other bearing. This enables usage of more powerful projectors, which provide more brilliant images, but are heavier a therefore not adapted to be hold in a hand. Even with a sophisticated bearing mechanism, moving the projector is restricted and may not allow projection of a visible image on every side of an object.

Preferably, the navigation system comprises a reference device for subsequent position measurement attached to the image projector and a position sensor for sensing the position of said reference, whereas a registration module is arranged in order to register the multidimensional data with the object. Registration may relate to any part of the human body. Subsequently, a registration method is applied to calculate a transformation (rigid, non-rigid) between the coordinate systems of the medical image data and the corresponding human body. Thus medical image data can be displayed spatially correct onto the patient's surface.

In a preferred embodiment, a discrimination module is arranged in order to determine patient specific structures (e.g. vessels, segments, tumours, etc.) based on multidimensional data, wherein the discrimination module is arranged to mark rendered image data in such a manner that patient specific structures are discriminable in the visible image projected on the object. Many of the tomography equipments used in hospitals already comprise a discrimination module, in order to distinguish between normal tissue and a tumor, for example.

Preferably, the rendering engine is based on a 3D virtual scene in which a virtual camera is deployed, wherein the virtual position of the virtual camera is defined by the relative placement of the projector and the object. The modelling software system comprises a virtual camera , wherein the properties (i.e. its position, orientation, focal length, distortion) of the virtual camera can be defined by software parameters. Furthermore, the parameters of the virtual camera correspond to the very same (inverse) parameters of the real projector device. Furthermore the pose parameters of the virtual camera system can be adjusted in real-time and according to the pose of the image projector relative to the target object. Thus, a virtual scene is rendered which can then be redirected to be projected through the image projection system..

An image projection method comprises the steps:

a) storage of multidimensional data of an object and/or models thereof in a database,

b) determination of positional data reflecting the relative placement of an image projector and the object,

c) generation of rendered image data based on positional data and multidimensional data of the object and/or models thereof, wherein the rendered image data is projected with the image projector as a visible image onto the surface of the object, and

d) generation of a refreshing event, in order to refresh the rendered image data.

According to this method, the internal structure and static or dynamic properties of an object may be easily displayed on the surface of an object from arbitrary viewpoints on the object. Therefore, the internal structure of the object may be easily investigated from various sides.

Preferably, refreshing events are generated at regular time intervals, preferably at least 20 times a second. Hence, the visible image displayed on the object is refreshed practically in real-time.

In a preferred embodiment, refreshing events are generated upon the detection of a change in the relative placement of the image projector and the object. Accordingly, in case the projector or the object is moved, the visible image displayed on the object is refreshed accordingly.

Preferably, multidimensional measurement data of an organ, a limb or any other part of a human body is acquired, wherein the visible image is projected onto the organ, the limb respectively the other part of a human body.

Other advantageous embodiments and combinations of features come out from the detailed description below and the totality of the claims.

**Brief description of the drawings**

The drawings used to explain the embodiments show:

Fig. 1    schematically an image projection system according to the invention;

Fig. 2    a handheld and navigated image projector;

Fig. 3    schematically the principles for rendering a visible image;

Fig. 4    the projector modelled as a virtual camera;

Fig. 5    the image overlay device calibration model with a navigated probe;

Fig. 6 a planar (2D) checkerboard and a 3D rigid model of the liver surface as test phantoms for evaluating the projection accuracy;

Fig. 7 navigated projection of the virtual checkerboard on the checkerboard plate image;

Fig. 8 navigated projection of the virtual surface grid on the surface of the liver phantom;

Fig. 9 reproduction error for ten different projections (different angles and distances) to evaluate overall system accuracy; and

Fig. 10 checkerboard image with reprojected grid corners and error vectors of a single image projection suitable for evaluation of the overall system accuracy.

In the figures, the same components are given the same reference symbols.

**Preferred embodiments**

I. Methods

A. System overview

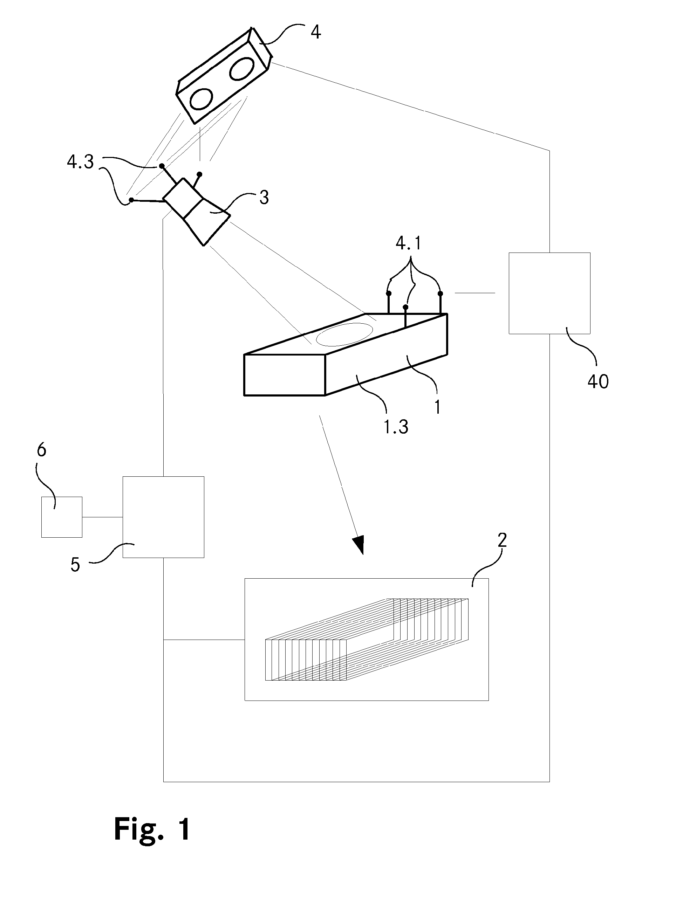

Fig. 1 shows schematically an object 1, a database 2 for storing multidimensional measurement data of the object 1, an image projector 3, which may also be called image overlay device, a position sensor 4, a position evaluation module 40 for determining positional data reflecting the relative placement of the image projector 3 and the object 1, a rendering engine 5 for generating rendered image data and a refresh module 6 for generating refreshing events.
The position evaluation module 40, the rendering engine 6, the refresh module 7 as well as other modules may be implemented as software modules running on a computer, for example on a PC with a touch-screen monitor.
The position sensor 4 may comprise infrared light-emitting diodes, such that light reflected from passive markers 4.1, 4.3 attached to a tool can be received by a pair of position sensors in order to determine the 3D position of the marker 4.1, 4.3. In case of several markers 4.1, 4.3, orientation of a tool may be determined as well (Vicra Camera, Northern Digital Inc, Canada). Instrument tracking is enabled by a navigation toolset composed of markers 4.1, 4.3 to be attached to calibration tools and existing surgical instruments (i.e. ultrasound knife, microwave ablation device).

B. Design of the image overlay device (also called image projector)

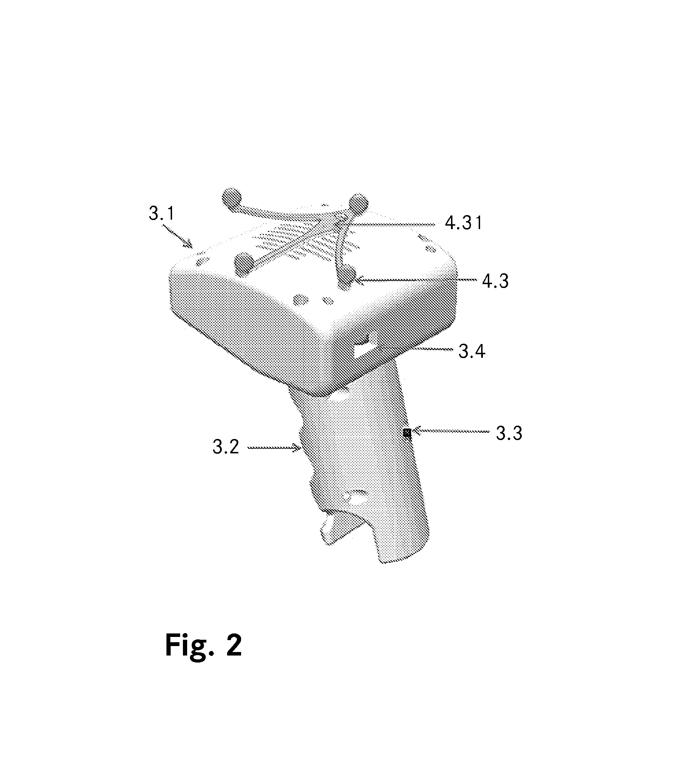

The image projector 3 or image overlay device incorporates a Microvision development kit (PicoP, Microvision Inc., USA) containing a portable RGB laser Pico Projector, a video processor and a micro-electro-mechanical system (MEMS) controller. The projector comprises a MEMS actuated deflection mirror (0 mirror: 1 mm) to reflect the combined RGB laser output, producing an active scan cone of 43.7˚ x 24.6˚. The projected images have a resolution of 848 x 480 pixels, a frame rate of 60Hz and a light intensity of 10 lumen. The absence of optical projection lenses and the matching of laser spot size growth rate to the image growth rate results in a projected image that is always in focus. The projector can therefore, be held at any distance from a projection surface. In order to maintain an appropriate image size and provide sufficient image intensity it is however, recommended to be used within a range of 0.1 to 0.7 m.
As depicted in Fig. 2, the image projector 3 or image overlay device comprises a protective housing, e.g. designed and manufactured using 3D printing rapid prototyping. A hand grip 3.2 makes the device easy to hold, even after a sterilized sleeve has been applied. The device receives the DVI/VGA video signal at the base of its handle from the navigation system's additional DVI output. A low noise fan for heat dissipation is integrated. An optical reference 4.31 with markers 4.3 is attached to the outer side of the device's housing and allows the image projector 3 to be tracked spatially by the navigation system. The reference and its placement on the housing are designed in a configuration that optimizes navigation visibility. The image projector 3 further comprises a projection window 3.1, a power on button 3.3 and a programming cable input 3.4 for development purposes.

C. Integration into a surgical environment

Sterilization in a surgical environment is achieved through the application of a standard transparent sterile drape (3M Steri-Drape) which covers the entire image overlay device and a portion of the attached cable. The sterile tracking reference 4.31 (suitable for autoclaving in standard hospital reprocessing) is then attached to the image overlay device on the outer side of the sterilized sleeve. Retro reflective marker spheres 4.3 (Brainlab AG, Germany) are attached to the tracking reference.

D. Image overlay device functionality

Prior to application of the image overlay device, the conventional steps of the navigation system application have to be conducted. Preoperatively, a 3D surface model consisting of patient specific structures (typically: vessels, segments and tumors) is reconstructed from patient tomography data. During the intervention instruments are calibrated, and the model is registered to the patient using anatomical landmark based rigid registration. Thereafter, the image overlay projector can be activated via the navigation system user interface.

The 3D pose (spatial position and orientation) of the image overlay device within the surgical scene is tracked by the navigation system in the coordinate system of the position sensor 4 ($^{IOD}T_{Sensor}$). Images for projection are rendered using a virtual camera 420, from within a virtual 3D scene, composed in the software control system.

The virtual camera 420 is defined by inversing the projector's projection parameters (e.g. distortion, focal length, opening angle, image aspect ratio) and the distance to the near and far clipping planes. The virtual camera's pose is defined as that of the calibrated projector in the sensor coordinate system, $^{CalProj}T_{sensor}$, given by:

$$^{CalProj}T_{Sensor} = {^{CalProj}T_{IOD}} \cdot {^{IOD}T_{Sensor}} \qquad \text{equation 1}$$

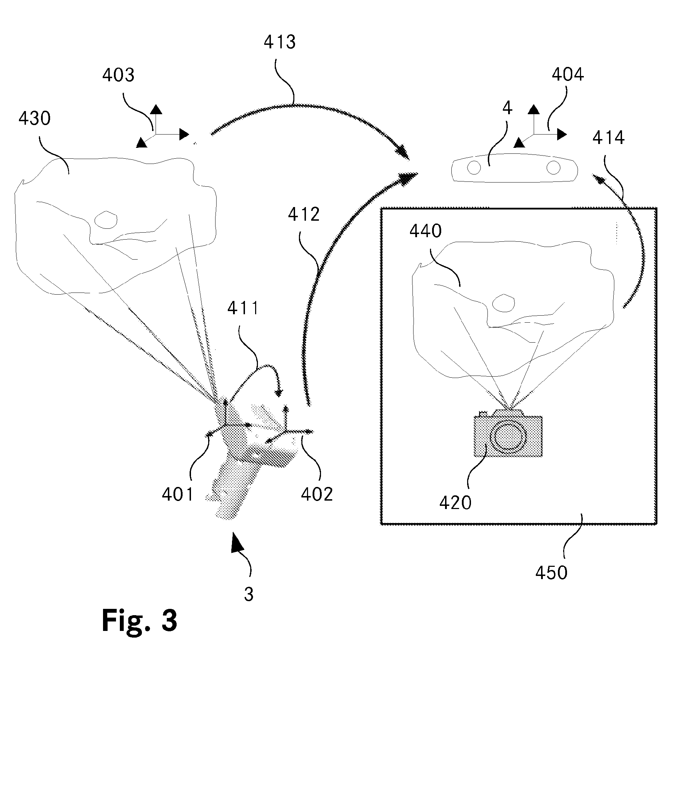

where $^{CalProj}T_{IOD}$ is the transformation relating the calibrated projection model to the image overlay device. The functional description of the image overlay device, also called image projector 3, is graphically displayed in Fig. 3. The following coordinate systems 401, 402, 403, 404 and transformations 411, 412, 413, 414 are displayed in Fig. 3:

401: *CalProj*  411: $^{CalProj}T_{IOD}$

402: *IOD*  412: $^{IOD}T_{sensor}$

403: *Patient*  413: $^{Patent}T_{Sensor}$

404: *Sensor*  414: $^{Model}T_{Sensor}$

Reference sign 430 refers to the real world object, whereas reference sign 440 refers to the virtual object in the virtual scene 450.

The rendered images are projected directly onto the surface of the object with an update rate equal to the maximum frame rate of the navigation system (e.g. 20 Hz).

To effectively project the images on to the target surface in a geometrically correct manner it is necessary to equate the camera model used for image capture of the virtual scene to the model of projection. To locate the pose of this model, the calibration transformation $^{CalProj}T_{IOD}$, mentioned above is calculated.

E. Projector calibration model

For the purpose of projection geometry calibration, projectors are often modeled as reverse pinhole cameras. For ease of coordinate system transformation, the projection transformation which specifies the relationship between the projected image and projector coordinate systems is expressed as a projection matrix solution. Using a common calibration camera model, the relationship between a point in space and its representation as an image pixel value is given by:

$$s\tilde{m} = A[R, T]\tilde{M} \qquad \text{equation 2}$$

The model relates the 2D image point $\tilde{m} = [u, v, l]$ expressed as an augmented matrix to the 3D real world point augmented matrix $\tilde{M} = [X, Y, Z, 1]$ where the extrinsic parameters R and T are the rotation and translation which relate the world coordinate system to the camera coordinate system and A is the intrinsic parameters matrix of the camera:

$$A = \begin{bmatrix} \alpha & \gamma & u_0 \\ 0 & \beta & v_0 \\ 0 & 0 & 1 \end{bmatrix}$$

equation 3

Here $(u_0, v_0)$ are the pixel coordinates of the principal point; $\alpha$ and $\beta$, the scale factors in the axes $u$ and $v$ respectively; and $\gamma$ is the skew of the two image axes.

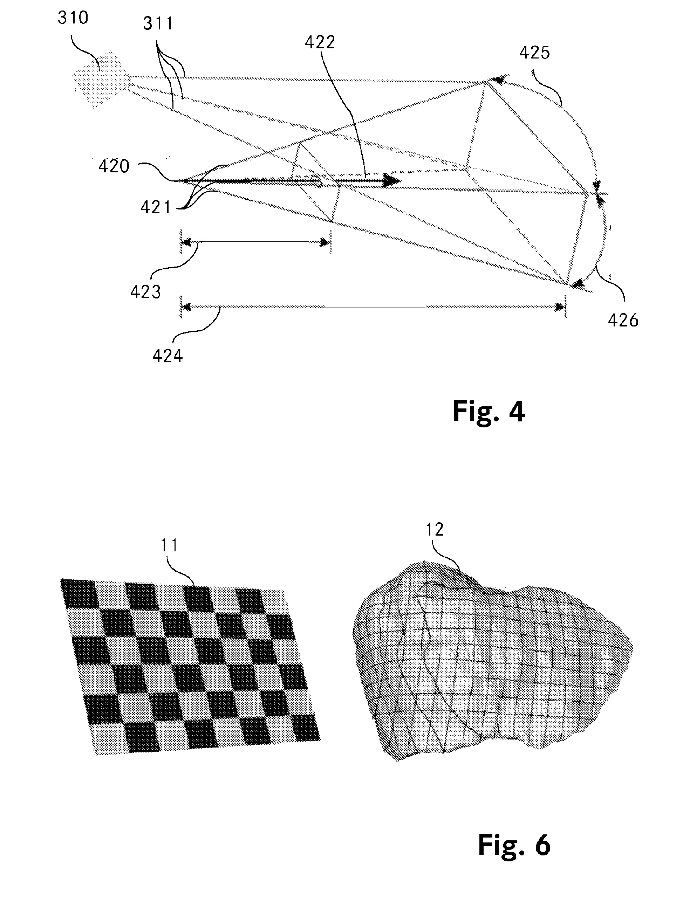

In Fig. 4, the reference sign 310 refers to the PicoP mirror of the projector. The reference sign 420 refers to the calibrated projection position of the Open Inventor™ camera. Reference sign 422 refers to the view direction. Reference sign 423 refers to the distance to the near clipping plane and reference sign 424 to the distance to the far clipping plane. The aspect ration is defined as x/y. Also defined in Fig. 4 is the width angle 425 and the height angle 426.

The virtual camera is modeled as an ideal pinhole camera and thus, its intrinsic parameters can be easily defined. The scale factors $\alpha$ and $\beta$ in both axes are said to be equal and the coordinate of the principle point $(u_o, v_o)$ is equal to the centre of the image. The camera has no skew between the two image axes ($\gamma$=1). A virtual camera model might not contain useful values for the distortion factor parameters and thus, distortion factors of the projector calculated using a known pinhole camera calibrated method, cannot be accounted for in the employed virtual image rendering method.

During application, the pose of the image overlay device is measured by the position sensor. To calculate the transformation between the calibrated projection model and the image overlay device, $^{CalProj}T_{IOD}$, calibration of the extrinsic projector parameters [$R$, $T$] are required.

Solving the pinhole camera model as described in equation 2 for the extrinsic camera parameters can be achieved through a closed form solution followed by a nonlinear refinement based on maximum likelihood criterion. 3D real world and 2D image point pairs $(M,m)_i$, are used for the calculation and can be acquired from a planar pattern, projected from a set of different orientations. To greatly reduce error in the calculation, a minimum number of such image planes are required from varying distances and orientations.

During operation, the pose of the image overlay device is tracked by the navigation camera. To calculate the transformation between the calibrated projection model and the image overlay device, $^{CalProj}T_{IOD}$, calibration of the extrinsic projector parameters [$R$, $T$] were required.

Solving the pinhole camera model as described in equation 1 for the extrinsic camera parameters can be achieved through a closed form solution followed by a nonlinear refinement based on maximum likelihood criterion. The 3D real world and 2D image point pairs $(M,m)_t$, required for the calculation can be acquired from a planar pattern, projected from at least two different orientations. To greatly reduce error in the calculation, at least 10 image planes are required and the angle between the camera image planes and the calibration pattern should be at least 45°.

The above calibration technique has been successfully applied in general camera calibration tasks as well as in the calibration of surgical augmented reality systems based on microscopes and was subsequently employed in the calibration of the image overlay device.

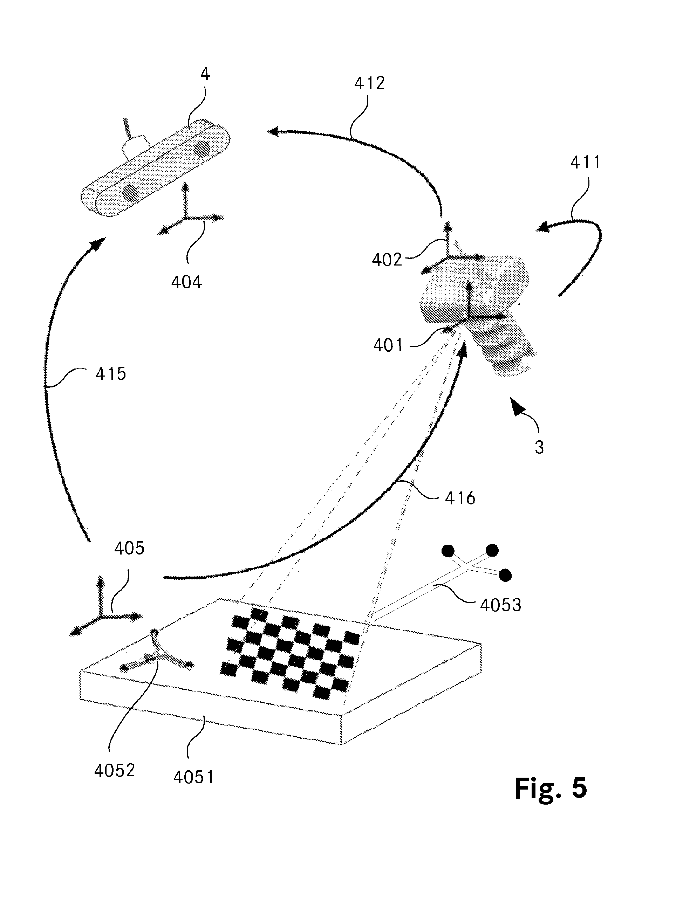

A rectangular grid is projected at ten different angles of orientations, onto a navigated projection plate 4051 whose position in space was measured using a pose measurement system 4052, as shown in Fig. 5, wherein the reference signs refer to the following coordinate systems 401, 402, 404, 405 and transformations 411, 412, 415, 416:

401: *CalProj*    411: $^{CalProj}T_{IOD}$

402: *IOD*    412: $^{IOD}T_{sensor}$

404: *Sensor*    416: $^{Plate}T_{CalProj}$

405: *Plate*    415: $^{Plate}T_{sensor}$

To obtain sufficient 3D information from the 2D projections, the projections were performed at angles greater than 45 degrees to the plate in the negative and positive x and y axes of the camera reference frame. The image overlay device was kept at a distance between 50 mm and 300 mm from the plane of projection to ensure that the projected images could be easily viewed and did not exceed the size of the plate 4051.

The 3D real world corner positions, $M_i$, of the projected grid patterns were digitized using a navigated probe 4053 in the coordinate system of the plate. The navigated probe 4053 itself was calibrated using the navigation system described above. The digitization of each point was performed three times and the positions averaged to reduce the effect of observer variability. In Fig. 5, a navigated probe 4053 and the 3D corner digitization setup is depicted.

Corresponding 2D image pixel coordinates of the checkerboard corners $m_i$ were extracted directly from the image for projection. For each projection, 7 x 5 point pairs of 2D pixel values $m_i$ with their respective real world 3D projection coordinates $M_i$ were acquired. The overall acquisition process resulted in 350 calibration point pairs.

With the collected point pairs $(M,m)_i$, and the intrinsic camera parameters of the virtual camera, $A$, equation 1 could be solved for extrinsic parameters $[R, T]$ via the Camera Calibration Toolbox for Matlab. As neither the projector nor the OpenGL camera incorporate an optical lens, the focal length was omitted from the calculation.

The extrinsic parameters $[R,T]$ of each projected image, $p$, were calculated and expressed as homogeneous matrices that define the transformations, relating the plate and the calibrated origins of projection, $(^{Plate}T_{CalProj})_p$, according to equation 5.

$$\left(^{Plate}T_{CalProj}\right)_p = \begin{bmatrix} R_p & T_p \\ 0 & 1 \end{bmatrix} \qquad \text{equation 5}$$

For each of the ten projections, the transformations from the position sensor to both the navigated projection plate and the image overlay device, $(^{Plate}T_{CalProj})_p$, and $(^{IOD}T_{sensor})_p$ respectively, were recorded and expressed as homogeneous matrices. The calibration transformations relating the calibrated origins of projection to the image overlay device, $(^{Plate}T_{CalProj})_p$, are then given by:

$$\left(^{CalProj}T_{IOD}\right)_p = \left(^{Plate}T_{CalProj}\right)_p^{-1} \cdot \left(^{Plate}T_{sensor}\right)_p \left(^{IOD}T_{sensor}\right)_p^{-1} \qquad \text{equation 4}$$

Fig.5 graphically depicts the calibration transformations.

Whilst the transformation $^{CalProj}T_{IOD}$ is a static parameter, error introduced during the calibration process results in variance across the set of matrices $(^{CalProj}T_{IOD})_p$, calculated from the 10 image projections. The transformation with the least reprojection error (as calculated by the Camera calibration Toolbox for Matlab) was selected to set the pose of the virtual camera and the others, discarded.

G. Accuracy evaluation

Accuracy analysis of the projection was performed both on a planar surface and on an irregularly shaped anatomical surface as shown in Fig. 6, which shows a planar checkerboard 11 and a liver model 12.

*Scenario1:* To determine the closed loop navigated projector accuracy, the resulting spatial displacement of projecting the above mentioned calibration checkerboard onto a print out of the checkerboard grid was identified. A planar CAD model of the calibration grid was constructed (Solidworks®, Dassault Systems, SolidWorks Corp., France) and integrated into the virtual scene within the navigation system. The calibration checkerboard print out was glued to a metallic plate and registered to its virtual model using the conventional landmarks based rigid registration approach of the navigation system.

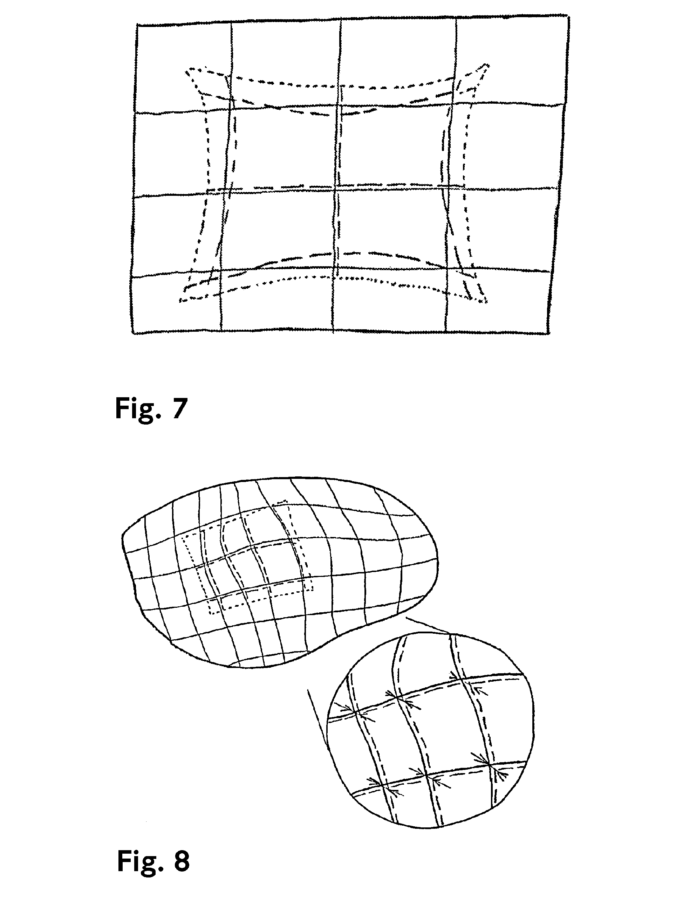

The checkerboard was projected by means of the image overlay module integrated into the navigation system. The displacement error of the projection of each checkerboard corner was calculated after digitizing the grid corner and the projected corner 3D positions with a navigated pointer tool. Data was collected for three different projection orientations. The first projection (orientation *a*) was conducted approximately normal to the model face while the second and third projections (orientations *a* and *b*) were performed at approximately $\pm45$ degrees to the model face. Fig. 7 shows schematically a projection of the virtual checkerboard onto the checkerboard plate image.

*Scenario 2*: To evaluate the projection accuracy on an anatomically relevant 3D surface, the above procedure was repeated with a rigid rapid prototyped model of a human liver with a superimposed 1 cm surface grid. The liver model was reconstructed from patient CT data. Fig. 8 shows schematically a projection of the virtual surface grid onto the surface of the liver phantom.

H. Clinical application evaluation

In an initial clinical assessment, a patient specific liver model (consisting of 3.000.000 triangles in 10 colors) was used to evaluate the usability and feasibility of the image overlay device in computer assisted liver interventions. Different projections of sub models depicting the various anatomical structures, i.e. tumors, resection planes and blood vessels, were performed on a real liver surface in a clinical scenario.

II. Results

The image overlay device was designed, manufactured and integrated into the current navigation system. Results of the projector calibration and an evaluation of the accuracy of the device projection are presented in the following sections in addition to a feasibility evaluation of the use of the device in a clinical application.

A. Calibration Results

The uncertainty corresponding to the calibrated extrinsic parameters, expressed as three times the standard deviations of the errors of estimation were calculated by the Camera Calibration Toolbox for Matlab and are presented in Table 1:

TABLE 1: ANALYSIS OF PROJECTION ERROR ()

|  | $R\ (\omega_x,\ \omega_y,\ \omega_z)$ | $T\ (X,Y,Z)$ mm |
|---|---|---|
| $E_{max}$ | (0.05, 0.06, 0.11) | (1.7,1.4, 7.4) |
| $E_{min}$ | (0.01, 0.01, 0.02) | (0.4, 0.7, 5.4) |
| $E_{mean}$ | (0.02, 0.03, 0.05) | (1.0, 1.1, 6.1) |

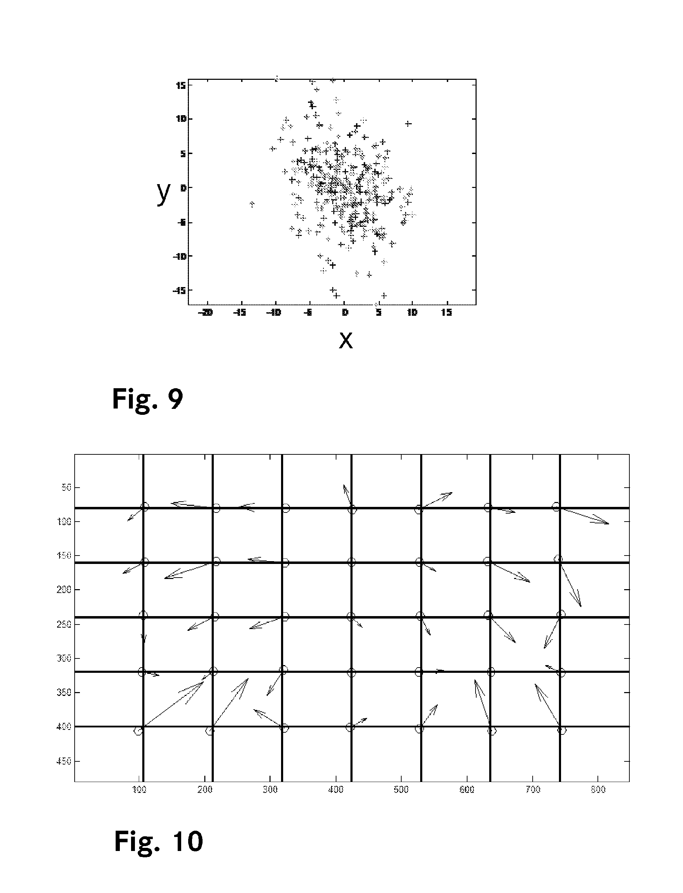

The error of reprojection, for each image projection, of the 3D points onto the image for projection using the stated intrinsic paramaters and the calculated extrinsic paramaters is graphically depicted below in Fig. 9. The projected image with the reprojected corner points and error vectors of a single projection overlayed is given in Fig. 10. In the Figure, the circles represent the positions of the reprojection error using a calibrated camera model. The arrows represent the normalized error vectors. The error is not actually as large as the arrow.

B. Accuracy evaluation results

The following spatial displacements were identified when projecting a checkerboard pattern on a planar surface (Scenario 1) and when projecting a 3D shaped pattern of a liver surface onto a 3D model (Scenario 2).

TABLE 2: ANALYSIS OF PROJECTION ERROR ()

|  | Scenario 1 Planar projection | Scenario 2 3D projection |
|---|---|---|
| $E_{max}$ | 3.59 mm | 4.99 mm |
| $E_{min}$ | 0.15 mm | 0.17 mm |
| $E_{mean}$ | 1.30 mm | 1.32 mm |
| σ | 0.74 mm | 0.87 mm |

C. Clinical application evaluation results

The system was successfully integrated in a surgical environment and deployed during surgery for liver metastasis ablation and liver resection. Prior to its use, the device was draped and the sterilized optical reference was attached. The image overlay device was successfully utilized by the surgeon to project sub models of the patient model onto the surface of the patient's liver.

The proposed image overlay device allows for visual guidance through the identification and visualisation of internal static or dynamiac information about an object directly onto the object's surface. Such image overlay is achieved with reduced complexity and setup time and with greater portability and workspace. For example, in surgery, the location of target structures such as metastases, can be projected onto the patient skin, in order to assist with the localisation of entry points for ablation or resection procedures. The overlay of vital structures also allows for intra-operative evaluation of risk and therefore promotes re-evaluation of surgical strategies.

The projection of navigated surgical tools can further improve spatial awareness and assists in promoting confidence in the guidance system as surgeons can instantly re-evaluate the accuracy of the projected images on available familiar objects.

The image overlay device requires no additional image-to-patient registration except that required in the navigation system. The image projection device can be connected to the navigation system and sterilized during the procedure. Possible applications include:

- Projections of 3D image data on the skin surface of the human body to localize anatomical structures underneath such as the display of fractures (i.e. arm, leg) to identify the relative pose of all fracture segments relative to each other.

- Visualization of 2D image data on the skin surface such as ultrasound imagery or fluoroscopy. Here the visualization in the same anatomical context can support the understanding of the image data by the physician.

- Projection of instrument guidance information onto the body of the patient in order to precisely guide the surgeon to a desired target. Here, a cross-hair visualization can be used to directly project the entry point of a biopsy needle on the human skin and in order to correctly guide the interventional physicist when avoiding sensitive anatomical structures.

It is believed that the presented work is a first step towards the application of the promising miniature projecting technology in medicine and surgery. Over time, many of the current drawbacks will be eliminated by more advanced technology and improved methodologies.

**Claims**

1. An image projection system, comprising:

    a) a navigation system (4, 4.1, 4.3, 40) for determining positional data reflecting the relative placement of an image projector (3) and an object (1),
    b) a database (2) for storing and retrieving multidimensional data of the object and/or models thereof, and
    c) a rendering engine (5) for generating rendered image data based on the multidimensional data and/or models thereof and the positional data, wherein the image projector (3) is arranged to project the rendered image data as a visible image (1.3) onto the surface of the object (1),

    **characterized in that**

    d) a refreshing module (6) is arranged for generating a refreshing event, in order to activate the rendering engine (5) and to refresh the rendered image data.

2. System according to claim 1, **characterized in that** a timer module generates refreshing events at regular time intervals, preferably at least 20 times a second.

3. System according to claim 1 or 2, **characterized in that** a position comparator module generates the refreshing event upon the detection of a change in the relative placement of the image projector and the object.

4. System according to one of claims 1 to 3, **characterized in that** a tomography equipment is arranged in order to acquire multidimensional measurement data of an organ, a limb or any other part of a human body, wherein the image projector is arranged to project the visible image on the organ, the limb respectively the other part of a human body measured by the tomography equipment.

5. System according to one of claims 1 to 4, **characterized in that** the image projector (3) is installed in a portable, handheld housing, which preferably is covered by a sterile drape.

6. System according to one of claims 1 to 5, **characterized in that** the navigation system (4, 4.1, 4.3, 40) comprises an reference device (4.3) for subsequent position measurement (4.3) attached to the image projector (3) and a position sensor (4) for sensing the position of said reference device (4.3), whereas a registration module is arranged in order to register the multidimensional data with the object.

7. System according to one of claims 4 to 6, **characterized in that** a discrimination module is arranged in order to determine patient specific structures (e.g. vessels, segments, tumours, etc.) based on multidimensional data, wherein the discrimination module is arranged to mark rendered image data in such a manner that patient specific structures are discriminable in the visible image projected on the object.

8. System according to one of claims 1 to 7, **characterized in that** the rendering engine is based on a 3D virtual scene in which a virtual camera is deployed, wherein the virtual position of the virtual camera is defined by the relative placement of the projector and the obj ect.

9. An image projection method, comprising the steps:

   a) storage of multidimensional data of an object in a database,
   b) determination of positional data reflecting the relative placement of an image projector and the object, and
   c) generation of rendered image data based on positional data and multidimensional data of the object, wherein based on the rendered image data a visible image (1.3) is projected onto the surface of the object with the image projector,

   **characterized by**

   d) generation of a refreshing event, in order to refresh the rendered image data.

10. Method according to claim 9, **characterized in that** refreshing events are generated at regular time intervals, preferably at least 20 times a second.

11. Method according to claim 9 or 10, **characterized in that** refreshing events are generated upon the detection of a change in the relative placement of the image projector and the object.

12. Method according to one of claims 9 to 11, **characterized in that** multidimensional measurement data of an organ, a limb or any other part of a human body is acquired, wherein the visible image is projected onto the organ, the limb respectively the other part of a human body.

**Fig. 1**

3.1
4.31
4.3
3.4
3.3
3.2

**Fig. 2**

**Fig. 3**

**Fig. 4**

**Fig. 6**

**Fig. 5**

Fig. 7

Fig. 8

**Fig. 9**

**Fig. 10**

Europäisches
Patentamt

European
Patent Office

Office européen
des brevets

**EUROPEAN SEARCH REPORT**

Application Number

EP 10 18 6627

| | **DOCUMENTS CONSIDERED TO BE RELEVANT** | | |
|---|---|---|---|
| Category | Citation of document with indication, where appropriate, of relevant passages | Relevant to claim | CLASSIFICATION OF THE APPLICATION (IPC) |
| X | US 2005/159759 A1 (HARBAUGH MARK [US] ET AL) 21 July 2005 (2005-07-21) * paragraphs [0047] - [0058]; claim 1; figure 1 * ----- | 1,6,9 | INV. A61B19/00 |
| X | WO 2008/032234 A1 (PHILIPS INTELLECTUAL PROPERTY [DE]; KONINKL PHILIPS ELECTRONICS NV [NL] 20 March 2008 (2008-03-20) * pages 4-6 * ----- | 1,7,9 | |
| A | US 6 314 311 B1 (WILLIAMS GILBERT T [US] ET AL) 6 November 2001 (2001-11-06) * the whole document * ----- | 1-12 | |
| A | US 2002/077533 A1 (BIEGER JOHANNES [DE] ET AL) 20 June 2002 (2002-06-20) * the whole document * ----- | 1-12 | |
| | | | TECHNICAL FIELDS SEARCHED (IPC) A61B |

The present search report has been drawn up for all claims

| Place of search | Date of completion of the search | Examiner |
|---|---|---|
| Munich | 28 February 2011 | Chopinaud, Marjorie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 18 6627

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2011

| Patent document cited in search report | | Publication date | Patent family member(s) | | Publication date |
|---|---|---|---|---|---|
| US 2005159759 | A1 | 21-07-2005 | AU | 2005206093 A1 | 04-08-2005 |
| | | | CA | 2553841 A1 | 04-08-2005 |
| | | | EP | 1706047 A1 | 04-10-2006 |
| | | | JP | 2007518521 T | 12-07-2007 |
| | | | WO | 2005070312 A1 | 04-08-2005 |
| WO 2008032234 | A1 | 20-03-2008 | AT | 479398 T | 15-09-2010 |
| | | | CN | 101511296 A | 19-08-2009 |
| | | | EP | 2066254 A1 | 10-06-2009 |
| | | | JP | 2010502343 T | 28-01-2010 |
| | | | US | 2010049037 A1 | 25-02-2010 |
| US 6314311 | B1 | 06-11-2001 | NONE | | |
| US 2002077533 | A1 | 20-06-2002 | DE | 10033723 C1 | 21-02-2002 |
| | | | JP | 2002102251 A | 09-04-2002 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020077533 A
- US 6314311 B